# EUROPEAN PATENT APPLICATION

(11) **EP 3 715 158 A1**
(43) Date of publication of application: **30.09.2020**
(21) Application number: 19165347.6
(22) Date of filing: 26.03.2019
(51) Int. Cl.: B60H 1/00, G01N 15/06, G01N 33/00, G01D 11/24, F16B 21/09

(54) **FLUID CIRCULATION HOUSING, ESPECIALLY TO MEASURE PARTICLES IN THE FLUID, AIR INTAKE AND HEATING, VENTILATING AND/OR AIR CONDITIONING UNIT COMPRISING SAID HOUSING**

(71) Applicant: Valeo Systemes Thermiques, 78320 Le Mesnil Saint Denis (FR)
(72) Inventor: POUYSEGUR, Serge, 78322 LE MESNIL SAINT-DENIS CEDEX (FR); PALETA, Michal, 78322 LE MESNIL SAINT-DENIS CEDEX (FR); LUER, Armin, 78322 LE MESNIL SAINT-DENIS CEDEX (FR); LAUCOURNET, Guillaume, 78322 LE MESNIL SAINT-DENIS CEDEX (FR); PAROUBEK, Pavel, 78322 LE MESNIL SAINT-DENIS CEDEX (FR); SCHNEIDER, Walther, 78322 LE MESNIL SAINT-DENIS CEDEX (FR)
(74) Representative: Tran, Chi-Hai

(57) **Abstract**

Fluid circulation housing comprising a fluid circulation body (2) and a fluid inlet tube (4) and/or a fluid outlet tube (6), said inlet tube (4) and/or said outlet tube (6) being in fluid communication with said body (2), said inlet tube (4) and/or said outlet tube (6) being oriented along a first direction (10), said housing (1) further comprising one or more fixing protrusions (12) so that said housing (1) can be attached to a supporting structure (14) according to a first translation along said first direction (10) followed by a second translation along a second direction (16) different from the first direction (10).

## Description

The invention relates to a fluid circulation housing, especially to measure particles in the fluid. It also relates to a heating, ventilating and/or air conditioning unit comprising said housing, the latter being configured to measure the particles in the air flowing through said unit and/or in the air outside of said unit. The invention may be used in a vehicle, for instance in a car.

Air intakes comprising a sensor measuring the particles in the air flowing there through are already known. The sensor is located inside the air intake, especially on flaps controlling the air flow.

However, such location has some drawbacks and there is a need to measure the particles in the air flow thanks to a component located outside.

The place available therefor is nevertheless very limited. Moreover, it may be wished to change the component without having to dismantle the entire air intake. The component has hence to be removable. More precisely, an operator needs to be able to remove and mount it again quickly without any tool. Indeed, manipulating a tool like a screw driver or the like may be impossible because of the encumbrance around the air intake which is to say under the dashboard.

Another issue is to efficiently feed the component with air potentially charged with particles so that said component can achieve a relevant measurement.

The invention aims at solving at least in part the above mentioned drawbacks and is related therefor to a fluid circulation housing comprising a fluid circulation body and a fluid inlet tube and/or a fluid outlet tube, said inlet tube and/or said outlet tube being in fluid communication with said body, said inlet tube and/or said outlet tube being oriented along a first direction, said housing further comprising one or more fixing protrusions so that said housing can be attached to a supporting structure according to a first translation along said first direction followed by a second translation along a second direction different from the first direction.

Thanks to such a double translation gesture, the housing can be easily attached even in overwhelmed places while simultaneously taking into account the orientation of the inlet and/or outlet tubes. The housing can hence be appropriately fed with fluid even if it is not located within the main stream but aside therefrom.

The invention can also comprise any of the following features taken individually or in any technically possible combination:
- said first and second directions are perpendicular,
- said protrusions extend sensibly perpendicularly to said first direction,
- said housing comprises a stop surface configured to block the housing against said supporting structure along said first direction,
- said stop surface is configured to guide said housing along said second direction when said stop surface is in contact with said supporting structure,
- said stop surface is configured to seal at least partially a trough hole provided in said supporting structure, said inlet and/or outlet tubes being intended to pass through said through hole,
- said inlet and/or outlet tubes are configured to be stopped by said supporting structure along said second direction,
- said circulation body comprises a large face,
- said stop surface extend ahead of said large face,
- said stop surface comprises a plate,
- said plate is located between said large face and said fixing protrusions,
- said protrusions extend from said large face,
- said protrusions extend from a periphery of said large face,
- said protrusions extend ahead of said large face,
- said housing comprises a support for said protrusions,
- said protrusions extends from said support,
- said support encloses said inlet and/or outlet tubes in a portion of surface of said large face,
- at least some of said protrusions extend laterally from said inlet and/or outlet tubes,
- at least some of said protrusions extend from said plate,
- at least some of said protrusions, especially said protrusions extending from said plate, have hook shape,
- a first branch of the hook extends along said first direction and a second branch of the hook extends along said second direction,
- said hooks are configured so that they define a stop against said supporting structure along said second direction,
- said first branch extends from said plate,
- said housing further comprises a snap component configured to block the housing when said protrusions are in a stop position after said second translation.

According to a first embodiment, said first direction is sensibly perpendicular to said large face of the circulation body.

According to another embodiment, said circulation body opens radially in said inlet and/or outlet tubes so that said first direction is sensibly parallel to said large face.

Whatever the applicable embodiment is, said inlet and/or outlet tubes extends preferably from said large face.

The invention also relates to a supporting structure on which a housing as described above is to be attached. It comprises an armature provided with said through hole. Said through hole is preferentially configured to further let said protrusions and/or said large face pass there through. Said armature is for instance sensibly planar.

The invention also relates to housing and a supporting structure as described above, preferably attached to one another.

The invention also relates to an air intake for a heating, ventilating and/or air conditioning unit, said air intake comprising an air flow channel and a fluid circulation housing, especially as described above, so that air flowing through said air flow channel further flows through said housing, said housing being configured to measure particles in said air, said air intake further comprising an interface, configured to drive the air between said channel and said housing, and an actuator system, located externally from said air flow channel, said interface being configured to support said housing with a lever arm so that said housing is located beyond said actuator system relative to said channel.

The invention can also comprise any of the following features taken individually or in any technically possible combination:
- said interface comprises a supporting structure, especially as described above, said housing being attached to said supporting structure,
- said air intake comprises a shelf defining partially said air flow channel,
- said interface is integral with said shelf,
- said air intake comprises one or more flaps configured to modulate the air circulating in said flow channel,
- said actuator system is configured to control said flap or flaps,
- said actuator system is fixed on a first lateral side of said channel,
- said flap comprises an articulation axis extending between said first lateral side and an opposite lateral side of said channel,
- said actuator system is located in an axial prolongation of said articulation axis,
- said interface is located in the vicinity of said actuator system.

The invention also relates to an heating, ventilating and/or air conditioning unit (HVAC unit) comprising a supporting structure, especially as described above, and a housing, especially as described above, attached to said supporting structure, said housing being configured to measure particles in the air exteriorly of the HVAC unit.

The invention can also comprise any of the following features taken individually or in any technically possible combination:
- said HVAC unit comprises a heat exchanger, especially a heating core, more especially an electrical heating core,
- said supporting structure is attached to said heat exchanger,
- said HVAC unit comprises an air circulation envelope configured to let the air flow trough said heat exchanger,
- said envelope comprise a mounting trap cover for said heat exchanger,
- said heat exchanger comprises a plate closing said trap cover,
- said supporting structure extends from said plate, exteriorly to said envelope,
- said supporting structure is perpendicular to said plate,
- said first direction is sensibly parallel to said plate.

The invention will be better understood thanks to the following description which is only indicative and which is not intended to limit said invention, accompanied with the following figures:
- figure 1 is schematic view showing in perspective a first embodiment of a housing and a supporting structure according to the invention,
- figure 2 is a schematic view showing in perspective a second embodiment of a housing according to the invention
- figure 3 is a schematic face view showing a supporting structure to be used with the housing of figure 2,
- figure 4 is a schematic view showing in perspective a third embodiment of a housing according to the invention,
- figure 5 is a schematic view showing in perspective a forth embodiment of a housing according to the invention
- figure 6 is another perspective view of the housing of figure 5 under another angle of view,
- figure 7 is another perspective view of the housing of figure 5 and 6 under another angle of view,
- figure 8 is partial perspective view of an air intake integrating the housing of figure 5 to 7,
- figure 9 is a perspective view showing some components of the air intake of figure 8,
- figure 10 is a cut view partially showing in perspective the air intake of figures 8 and 9,
- figure 11 is a perspective view partially showing an HVAC unit integrating the housing of figure 5 to 7 according to another embodiment of the invention,
- figure 12 is a perspective view showing under another angle of view an area of the HVAC unit of figure 11, said area integrating the housing.

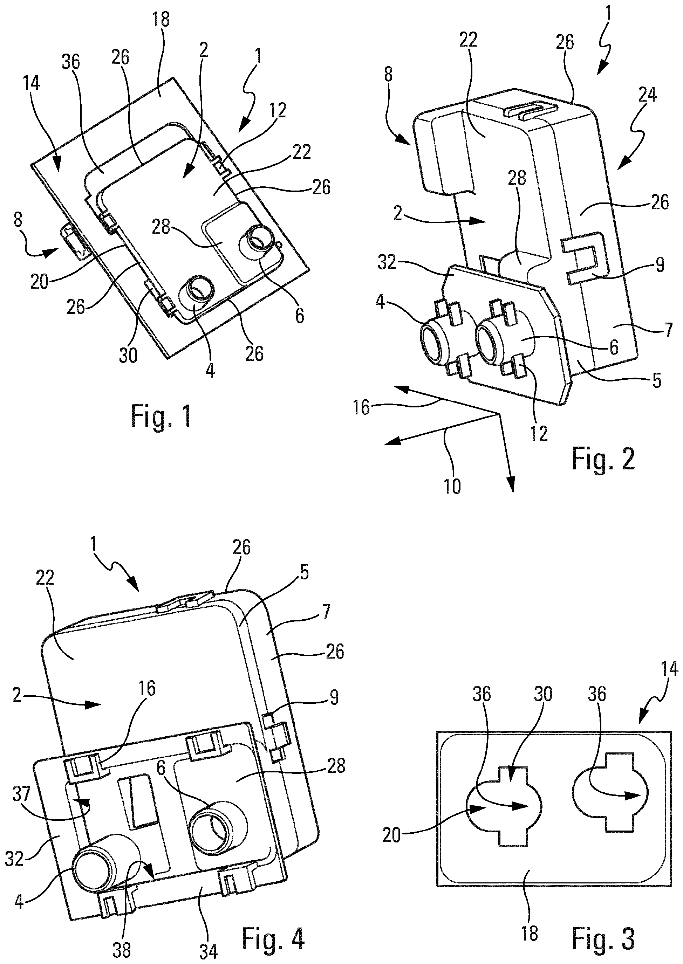

As shown on figures 1, 2 and 4 to 7, the invention first relates to a fluid circulation housing 1. It comprises a fluid circulation body 2 and a fluid inlet tube 4 and/or a fluid outlet tube 6. Said inlet tube 4 and/or said outlet tube 6 are in fluid communication with said body 2.

Said fluid circulation body 2 preferentially has a large face 22. Here, more precisely, said fluid circulation body 2 has a general cuboid shape with two opposite large faces 22, 24 and lateral faces 26 joining said opposite large faces 22, 24.

The fluid is preferentially air and said housing is for instance configured to measure particles in the air flowing there through. While not shown, the circulation body comprises internally a channel along which air flows between the inlet tube 4 and the outlet tube 6. The channel has for instance a length longer than a distance between the inlet tube 4 and the outlet tube 6. It may have a U configuration.

The circulation body 2 further comprises a particles sensor located along the channel. The housing 1 further comprises a connector 8 providing an electrical supply to the sensor and connecting the latter to an external data communication network. Said connector 8 is for instance located on one of said large face 22, 24 or one of said lateral faces 26 of said fluid circulation body 2.

Said circulation body 2 is preferentially in two parts and comprise a first shell 5 provided with the inlet and the outlet tubes 4, 6 and second shell 7 forming a cover. The first and second shells are for instance linked by clips 9 located on the lateral faces 26 of the circulation body 2. The circulation body 2 may be made of plastic, especially by molding.

Said inlet tube 4 and/or said outlet tube 6 are oriented at least along a first direction 10. The inlet and/or outlet tubes 4, 6 have for instance a cylindrical shape, especially with a rounded cross section, more especially with an oval or circular cross section, and said first direction 10 is defined by a longitudinal axis of said cylindrical shape.

Said inlet and/or outlet tubes 4, 6 are linked to a first 22 of said large face. More precisely, here, said large face 22 has a two longitudinal sides joined by two lateral sides, said longitudinal sides and said laterals side being joined by rounded corners. Said inlet tube 4 and said outlet tubes 6 are located in the vicinity of two adjacent of said corners, linked to a common lateral side of said large face 22.

Said inlet and/or outlet tubes 4, 6 extend from said large face 22. Here said inlet tube 4 extends directly from a planar portion of said large face 22 while said outlet tubes 6 extends from an outlet box 28 provided on said planar portion of said large face 2. Said outlet box 28 has in itself a cuboid shape. Said outlet box 28 extends for instance until the adjacent longitudinal and lateral sides of said large face 22.

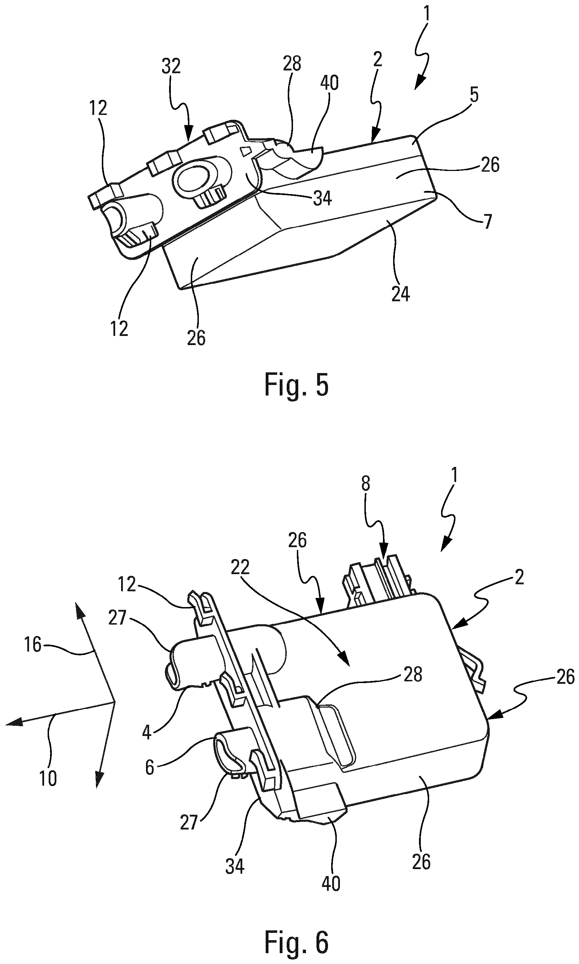

Said housing further comprises one or more fixing protrusions 12. Said fixing protrusions 12 are for instance defined by tabs. Said tabs extend sensibly perpendicularly to said first direction 10. They have here a U cross section. All or some of said fixing protrusions 12 may also have a hook shape as in figures 5 to 7.

According to a first aspect of the invention, said housing 1 is configured to be attached to a supporting structure 14 (see especially figures 1 and 3) thanks to said fixing protrusion 12 according to a first translation along said first direction 10 (arrow 10 only shown on figures 2 and 6) followed by a second translation along a second direction 16 (arrow 16 only shown on figures 2 and 6) different from the first direction 10. Said first and second directions 10, 16 are preferentially perpendicular.

Said supporting structure 14 comprises at least one through hole 20 provided here in an sensibly planar armature 18. Said inlet and/or outlet tubes 4, 6 are intended to pass through said through hole 20. Said through hole 20 is further configured to let said fixing protrusions 12 pass there through.

After the first translation, said fluid circulation body 2 is located on one first side of said supporting structure 14, especially of said armature 18, and the fixing protrusion 12 are located beyond an opposite parallel second side of the supporting structure 14, especially of said armature 18, said fixing protrusion 12 still being in front of said through hole our holes 20. Both said first and second sides are for instance sensibly planar. After said second translation, said fluid circulation body 2 is still located on said first side of said supporting structure 14 while said fixing protrusions 12 are then located in front of said second side.

As a result of such successive translations, the housing 1 is stopped by said fixing protrusion 12 against said supporting structure 14 along a direction opposite to said first direction 10 and cannot be removed unless by an inverse double translations movement.

The housing 1 is preferentially configured to be stopped after said second translation against said supporting structure 14 along said second directions 16 and/or a third direction perpendicular to both first and second directions 10, 16. This result can be obtained for instance trough shape cooperation between said housing 1 and said supporting structure 14, more precisely with an edge of said through hole or holes 20. The housing 1 is further preferentially configured to be guided by said supporting structure 14 during said second translation, especially by shape cooperation, more especially by said edge of the trough hole or holes 20, as will be seen below according to the various embodiments which are presented.

According to the embodiments shown on figures 1 to 4, said first direction 10 is sensibly perpendicular to said large face 22 of said body 2. Moreover said inlet and outlet tubes 4, 6 are sensibly linear which is to say that said first and/or second outlets tubes 4, 6 are sensibly perpendicular to said large face 22.

Said outlet tube 6 extends from a face of said outlet box 28 which is parallel to the planar portion of said large face 22. It is shifted from said inlet tube 4 according said third direction.

According more precisely to the embodiment shown on figure 1, said protrusions 12 extend from said large face 22, especially from a periphery of and/or sensibly in parallel to said large face 22.

In this embodiment, there is provided one through hole 20. Said through hole 20 is further configured to let said large face 22 pass there through so that the above mentioned double translation gesture can be achieved. To guide and stop the housing 1 in relation with said second translation, a contour of the through hole 20 is preferentially matching with a contour of said large face 22 to which is added a gap 36 corresponding to the stroke of said housing 1 during said second translation. Said contour of the through hole further has notches 30 to match with the fixing protrusions 12, here having a tab shape. The housing has for instance four such tabs, a pair thereof being located along two opposite of said longitudinal sides of said large face 22.

According more precisely to the embodiments shown on figure 2 to 4, said protrusions 12 extend ahead of a sensibly parallel to said large face 22.

Said housing 1 comprises a stop surface 32 blocking the housing 1 against said supporting structure 14 along said first direction 10 both after said first translation and in use after said second translation. Said stop surface 32 extends ahead of said body large face 22.

It is preferentially configured to guide said housing 1 along said second direction 16 when said stop surface 32 is in contact with said supporting structure 14.

More preferentially, said stop surface 32 is configured to seal at least partially said trough hole or through holes 20 provided in said supporting structure 14.

Said stop surface 32 comprises for instance a plate 34 located between said protrusions 12 and said large face 22. Said plate 34 is parallel to said large face 22 and to said tabs 12.

Said inlet and/or outlet tubes 4, 6 are configured to be stopped by said supporting structure 14 in said through hole or through holes 20 along said second direction 16 and/or said third direction.

According to the embodiment of figure 2, said protrusions 12, here having a tab shape, extend laterally, especially radially, from said inlet and/or outlet tubes 4, 6. The housing has for instance four such tabs 12. A first pair is located on the inlet tube 4, the tabs 12 of said first pair being diametrically opposed along said third direction. A second pair is located on the outlet tube 6, the tabs of said second pair being diametrically opposed along said third direction.

Said plate 34 extends from said inlet and/or outlet tubes 4, 6 in said second and third direction. It is for instance sensibly rectangular with chamfered corners.

As shown in figure 3, in the same embodiment, the supporting structure 14 comprises two through holes 20. To guide and stop the housing in relation with said second translation, a contour of each of said through holes 20 is preferentially defined by the contour of said inlet and outlet tubes 4, 6 to which is added a gap 36 corresponding to the stroke of said housing 1 during said second translation. As a consequence, said through holes 20 sensibly have an oval shape. Said contour of the through holes further has notches 30 to match with the fixing protrusions 12.

Such embodiment has the advantage to limit the size of the holes to be provided in the supporting structure compared to the previous embodiment.

As shown on figure 4, in an intermediate embodiment, said housing comprises a support 37 for said protrusions 12 and said protrusions 12 extends from said support 37 ahead of said large face 22. Said support 37 comprises an annular wall extending from said large face 22. One side 38 of the wall, called bottom side, is in the continuity of one of the lateral side 26 of the circulation body 2 and two others, contiguous to the bottom side 38, are in the continuity of two others of said lateral sides 26.

Said plate 34 defining a stop surface 32, extends from said support 37 in said second and third directions to define a ring extending outwardly from said wall 38.

Said support 36 encloses said inlet and/or outlet tubes 4, 6 in a portion of surface of said body large face 22, especially a portion including said outlet box 28.

In such embodiment the supporting structure (not shown) has a single through hole. To guide and stop the housing in relation with said second translation, a contour of said through hole is preferentially matching with a contour of said support 37 to which is added a gap corresponding to the stroke of said housing 1 during said second translation. Said contour of the through hole further has notches to match with the fixing protrusions 12.

The housing 1 has for instance four tabs 12 extending by pair from two opposite sides of the wall, one pair thereof extending from said bottom side 38.

According to the embodiment shown on figures 5 to 12, said first direction 10 is sensibly parallel to said large face 22 of said body 2. To that end, here, said inlet and/or outlet tubes 4, 6 are located above said large face 22 and extend in parallel thereof so that free ends of said inlet and/or outlet tubes 4, 6 are located beyond one of said lateral face 26 of said circulation body 2, called adjacent lateral face.

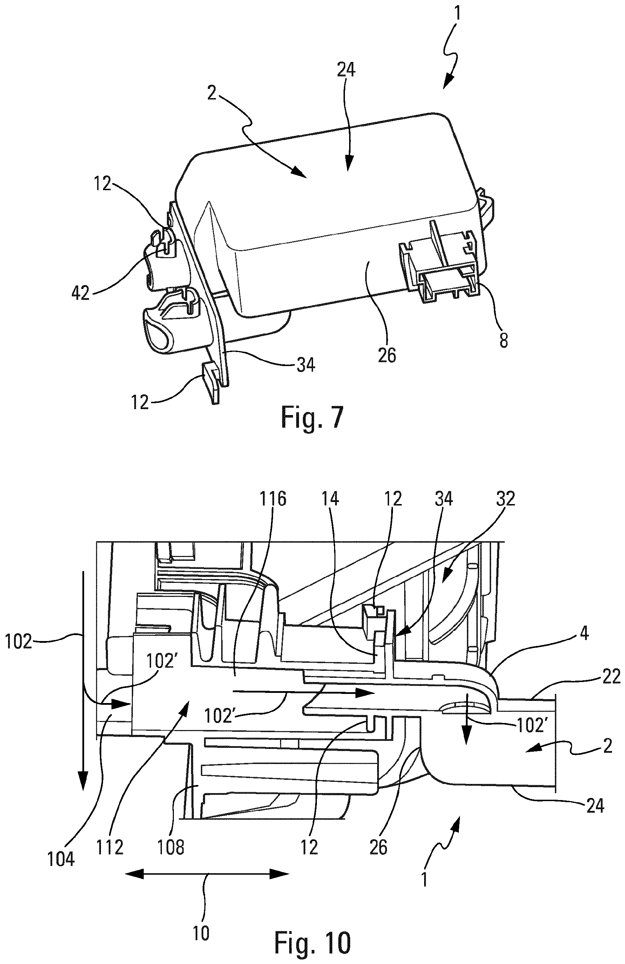

Said circulation body 2, especially said large face 22, opens radially in said inlet tubes 4 (see figure 10). Said outlet tube 6 extends from a lateral face of said outlet box 28.

The free end of said outlet tube 6 is closer from said body 2 than the free end of the inlet tube 4. Said inlet and outlet tubes 4, 6 are located along the same level according said third direction.

As can be seen on figures 5 to 7, said inlet and/or outlet tubes 4, 6 have a flatten cross section with a longer dimension along said second direction and smaller dimension along said third direction providing each tube with two parallel large sides linked by small sides formed by a radius. The free ends of the inlet and/or outlet tubes 4, 6 have a first part parallel to the adjacent lateral face 26 and another cut part 27 located on one of their radius, said radius being cut according to a plane comprising said third direction. On each inlet and/or outlet tubes 4, 6, the cut radius 27 of their free ends are turned towards two opposite lateral faces 26 of said circulation body.

In these embodiment too, said housing 1 comprises a stop surface 32 blocking the housing 1 against said supporting structure 14 along said first direction 10 both after said first translation and in use after said second translation. It is preferentially configured to guide said housing 1 along said second direction 16 when said stop surface 32 is in contact with said supporting structure 14.

More preferentially, said stop surface 32 is configured to seal at least partially said trough hole or through holes 20 provided in said supporting structure 14.

Said stop surface 32 comprises for instance a plate 34. Here, said stop surface 32, especially said plate 34, extends transversally, for instance perpendicularly, to said large face 22. Said plate 34 is located between the protrusions 12 and the adjacent lateral face 26.

In this embodiment, the housing 1 has for instance two protrusions in the shape of tabs 12, one of them being located on said inlet tube 4 and the other on the outlet tube 6. They have here an elongated U cross section. A slit 42 is provided on two opposite lateral sides thereof.

Said protrusions 12 also comprise hooks. There are here three hooks. They are extending from the plate 34. They are located along an upper edge of said plate 34 while the tabs 12 are located beneath the inlet and outlet tubes 4, 6. The tabs and the hooks are shifted along said second direction 16.

Said hooks have for instance an L shape, a first branch of the hook extending along said first direction 10 and a second branch of the hook extending along said second direction 16. Said first branch extends from said plate 34. Said hooks are configured so that they define a stop against said supporting structure 14 along said second direction 16.

The use of hooks is also possible on the previous embodiments. More generally, in whatever embodiments, the protrusion can comprise only tabs, only hooks, or a combination of tabs and hooks without departing from the invention.

Said housing further comprises a snap component 40 configured to block the housing 1 when said protrusions 12 are in a stop position after said second translation. Said snap component 40 is located on one of the lateral face 26 of the circulation body, for instance the lateral side 26 opposite to the lateral side 26 provided with the connector 8, with a gap there between. Said snap component 40 is here defined by a flexible tab articulated on said plate 34 and maybe molded therewith.

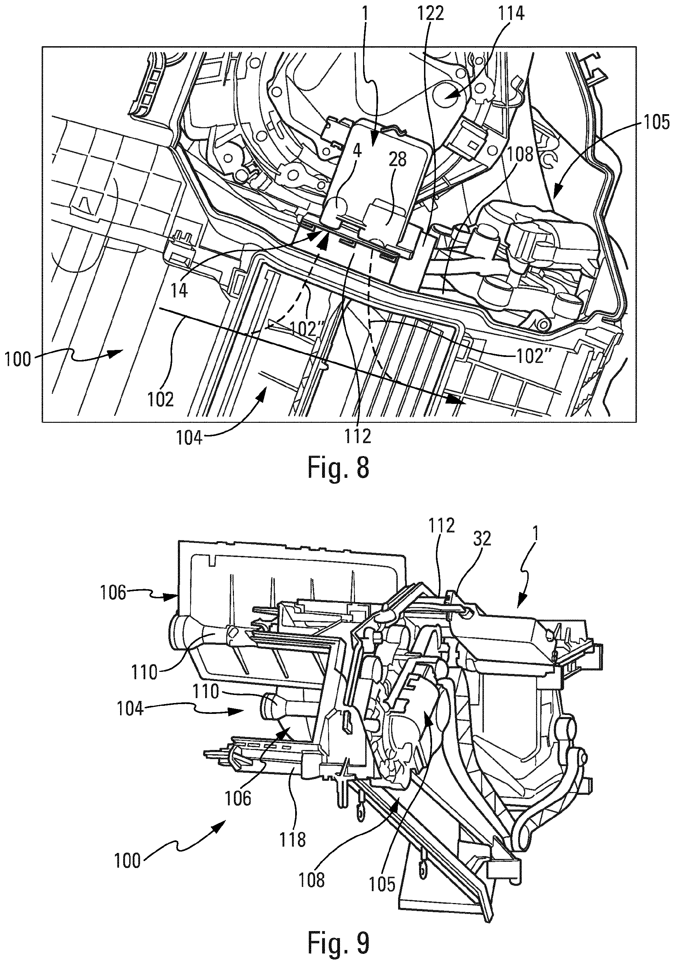

As can be better seen on figures 8 and 9, the invention also relates to an air intake 100 for a Heating, Ventilating and/or Air Conditioning (HVAC) unit.

Said air intake 100 comprises a supporting structure 14, especially as described above, and a fluid circulation housing 1, especially as described above. In other words, said housing 1 is attached to said supporting structure 14, especially in a removable way. More precisely, said housing 1 is attached to said supporting structure 14 so that air flowing through said air intake, as illustrated by the arrow 102, further flows through said housing as illustrated by the arrows 102', 102".

Said air intake 100 comprises an air flow channel 104 and one or more flaps 106 configured to modulate the air flow according to its or their position.

Said air intake 100 further comprises an actuator system 105 configured to control the position of said flap or flaps 106. Said actuator system 105 is located externally on a first lateral side 108 of said channel 104. Said actuator system 105 comprises for instance an electrical motor and/or a transmission mechanism to drive said flap or flaps 106.

Said flap or flaps 106 comprise an articulation axis 110 extending between said first lateral side 108 and an opposite lateral side (not visible on the figures) of said channel 104. Said actuator system 105 is located in an axial prolongation of said articulation axis 110.

According to another aspect of the invention the air intake further comprise an interface 112 configured to drive the air between said channel 104 and said housing 1. Moreover, said interface 112 is configured to support said housing 1 with a lever arm so that said housing is located beyond said actuator system 105 relative to said channel 104. Said lever arm is here oriented along said first direction 10.

Thanks to the interface 112, the housing 1 can be placed on the same side of the channel 104 as the actuator system 105 which optimizes the encumbrance of the air intake, especially when said actuator system 105 and said housing 1 are placed above an air flow generator 114, like a pulser, of said air intake.

Said interface 112 is preferentially located in the vicinity of said actuator system 105.

In an advantageous embodiment, said air intake comprises a shelf 118 defining partially said air flow channel 104. Said interface 112 is here integral with said shelf 118.

In an alternative embodiment, said interface 112 is attached in removable way to said channel 104, especially said shelf 118, so that a same air intake can be used with or without said interface 112 when said housing 1 is not needed.

Said interface 112 comprises here said supporting structure 14 on which said housing 1 is attached according to the double translation gesture explained above. However according to the second aspect of the invention, the housing 1 and its supporting structure can be configured to be attached to one another in every other ways.

The interface 112 has two large parallel faces linked by lateral faces. A first lateral face is defined by the lateral side 108 of the air flow channel 104, said lateral side being provided by apertures trough which air circulates between the interface 112 and the air channel 104. An opposite lateral side is defined by said supporting structure 14 through which, as already said, air circulates in said inlet and/or outlet tubes 4, 6 between said interface 112 and said housing 1. An other rounded lateral side of the interface 112 link the first lateral side and the opposite one as well as the two large parallel faces thereof. On the opposite side, the interface is provided with a locking surface 122 extending beyond said supporting structure 14 along said first direction 10. Said locking surface 122 is provided with a notch in which said snap component 40 is locked when said protrusions 12 are in said stop position after said second translation.

Said snap component 40 rides on the surface 122 perpendicular to the first lateral side 108 of the air intake 100 during the second axial movement in direction 16. The complete stroke is achieved when said inlet and outlet tubes 4, 6, come in contact with the inner contour of said trough hole 20. Then, said snap component 40 springs back into the empty space created by the notch provided in said locking surface 122 thereby locking said housing 1 into final position. For service a simple push of the mechanics by a finger on the snap component 40 is enough to disengage it from its end position and pull the housing 1 in the opposite direction.

As can be best seen on figure 10, the interface 112 further comprises inlet and outlet internal channels 116 to guide the air into the inlet tube 4 and out from the outlet tube 6. All or part of said fixing protrusions 12 can be inside said internal channels 116 and/or outside said internal channels 112. Here, the hooks are located outside the internal channels 116. The tabs are located inside the internal channels 116.

However, the fluid circulation housing according to the invention can be located elsewhere in a HVAC unit to measure particles in the air stream of the HVAC unit, for example between a blower and an evaporator of the HVAC unit.

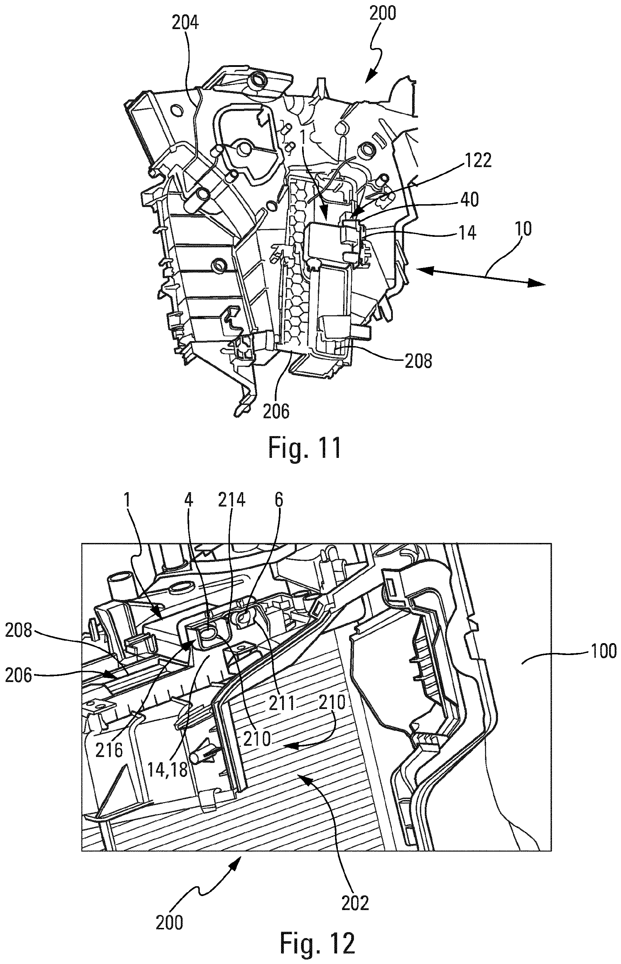

As illustrated on figures 11 and 12, the invention also relates to an HVAC unit 200 comprising a supporting structure 14, especially as described above, and a housing 1, especially as described above. Said housing 1 is attached to said supporting structure 14, especially in removable way.

According to a third aspect of the invention, said housing 1 is configured to measure particles in the air exteriorly of the HVAC unit.

While there is a first interest to measure particles in the air flowing within the HVAC unit before it enters the vehicle compartment, there is also a need to measure particles in the air in the vehicle compartment itself. A first solution in this view is to locate a particle sensor somewhere in the vehicle compartment. Nevertheless, there is also a need to hide such a particle sensor.

It has been found by the applicant that the air quality under the dashboard is sensibly the same as the air quality of the vehicle compartment. As a consequence, to fix the housing 1 to an HVAC unit means that the housing 1, as the HVAC unit itself, will be hidden under the dashboard. Simultaneously, as explained above, to provide a measure of the particles level exteriorly of the HVAC which is to say of the particles in the air under the dashboard will provide a reliable measure of the particles level in the vehicle compartment. Moreover, to integrate the housing 1 in the HVAC unit gives the possibility to avoid fixing the housing 1 separately.

In case of a supporting structure 14 and a housing 1 as described in relation with the embodiments illustrated on the drawings, said housing 1 is attached to the supporting structure according to the double translation gesture explained above. However according to the third aspect of the invention, the housing 1 and its supporting structure 14 can be configured to be attached to one another in every other ways.

Said HVAC unit 200 comprises for instance an air intake 100 as described above. In other words, said HVAC unit may comprise a first housing 1 associated to the air intake 100 to measure particles in the air flowing inside the HVAC unit and another one to measure particles in the air exteriorly of the HVAC unit.

Said HVAC unit 200 further comprises an air circulation envelope 204. Said HVAC unit may also comprise a heat exchanger 202, especially a heating core. It mays also comprise another heat exchanger as an evaporator (not shown). Said air circulation envelope 204 is configured to let the air flow trough said heat exchanger 202, eventually according to the position of a mixing flap (not shown).

Said envelop 204 comprises a mounting cover 206 for said heat exchanger 202. Said mounting trap cover 206 is here located on a lateral side of said HVAC unit 200.

Said heat exchanger 202 comprises a plate 208 closing said cover 206. Said plate 208 is here located on a lateral side of a core 210 of the heat exchanger 202.

Said supporting structure 14, especially said armature 18, is preferentially attached to said heat exchanger 202. More precisely said supporting structure 14 extends from said plate 208, exteriorly to said envelop 204. In other words, said supporting structure 14 is integral with said closing plate 208.

The armature 18 of said supporting structure 14 is for instance perpendicular to said closing plate 208. Said first direction 10 of the double translation gesture is here sensibly parallel to said closing plate 208.

Said inlet and/or outlet tubes 4, 6 opens in the air outside the HVAC unit 200 through apertures corresponding to said through holes of said supporting structure 14.

Said inlet tube 4 opens through a first 210 of said apertures and said outlet tube opens through a second 212 of said apertures. Said first aperture and said second aperture are separated by a flange 214. Said flange 214 is here part of a duct 216 extending from said armature 18.

In said embodiment, said locking surface 122 cooperating with said snap component 40 of the housing 1 is provided on top of a finger extending perpendicularly from said closing plate 208.

## Claims

1. Fluid circulation housing comprising a fluid circulation body (2) and a fluid inlet tube (4) and/or a fluid outlet tube (6), said inlet tube (4) and/or said outlet tube (6) being in fluid communication with said body (2), said inlet tube (4) and/or said outlet tube (6) being oriented along a first direction (10), said housing (1) further comprising one or more fixing protrusions (12) so that said housing (1) can be attached to a supporting structure (14) according to a first translation along said first direction (10) followed by a second translation along a second direction (16) different from the first direction (10).

2. Housing according to claim 1 wherein at least some of said protrusions (12) extend laterally from said inlet and/or outlet tubes (4, 6).

3. Housing according to any of claims 1 or 2 wherein said housing (1) comprises a stop surface (32) configured to block the housing (1) against said supporting structure (14) along said first direction (10).

4. Housing according to claim 3 wherein said stop surface (32) is configured to guide said housing (1) along said second direction (16) when said stop surface (32) is in contact with said supporting structure (14).

5. Housing according to any of claims 3 or 4 wherein said stop surface (32) is configured to seal at least partially a trough hole (20) provided in said supporting structure (14), said inlet and/or outlet tubes (4, 6) being intended to pass through said through hole (20).

6. Housing according to any of claims 3 to 5 wherein said inlet and/or outlet tubes (4, 6) are configured to be stopped by said supporting structure (14) along said second direction (16).

7. Housing according to any of claims 3 to 6 wherein said stop surface (32) comprises a plate (34) and at least some of said protrusions (12) extend from said plate (34).

8. Housing according to claim 7 wherein said protrusions (12) extending from said plate (34) have a hook shape, said hooks being configured so that they define a stop against said supporting structure (14) along said second direction (16).

9. Housing according to any of the preceding claims wherein said circulation body (2) opens radially in said inlet and/or outlet tubes (4,6) so that said first direction (10) is sensibly parallel to a large face (22) of said circulation body (2).

10. Assembly of a housing (1) according to any of the preceding claims and said supporting structure (14).
